# EUROPEAN PATENT APPLICATION

(11) **EP 3 530 315 A1**
(43) Date of publication of application: **28.08.2019**
(21) Application number: 18171581.4
(22) Date of filing: 09.05.2018
(51) Int. Cl.: A61N 1/372, A61B 17/22, A61N 1/05

(54) **A MEDICAL IMPLANT SYSTEM FOR RECAPTURING/EXPLANTING AN IMPLANT**

(30) Priority: 22.02.2018 US 201862633626 P
(71) Applicant: BIOTRONIK SE & Co. KG, 12359 Berlin (DE)
(72) Inventor: Taff, Brian M., Portland, OR 97217 (US); Kibler, Andrew B., Lake Oswego, OR 97035 (US); Whittington, R. Hollis, Portland, OR 97202 (US); Muessig, Dirk, West Linn, OR 97068 (US); Stotts, Larry, Tigard, OR 97224 (US)
(74) Representative: Engelhardt, Björn

(57) **Abstract**

The disclosure relates to a medical implant system (1), comprising: an implant (2) that is implantable into a patient, and a catheter (3) configured for explanting the implant (2). The catheter (3) and/or the implant (2) is configured to measure a distance (D) between a tip (30) of the catheter (3) and the implant (2). The system (1) is configured to generate an output signal indicative of said distance (D). In another embodiment, the system (1) comprises a sensor element (22) which is configured to measure at least one physical quantity indicative of a local anatomical environment of the implant (2). Also, methods for recapturing and/or explanting an implanted implant (2) are provided.

## Description

The disclosure relates to medical implant systems and methods for recapturing/explanting an implant.

The successful reengagement of a mechanical linkage between an implanted implant such as a leadless pacemaker and its affiliated delivery/explantation tooling (e.g. catheter) presents a significant procedural challenge. Much of this challenge arises due to the tortuous path the tooling must navigate as it is routed from the femoral access, through the IVC (vena cava inferior), and into the right side of the patient's heart. When paired with the available range of motion enabled by articulations within the delivery/explantation tooling and the fact that the implanted implant (e.g. a leadless pacer) may reside in orientations not initially aligned with the approaching catheter, any and all aids to recapture and re-engagement procedures prove beneficial. In the context of chronic implantation, the implant may additionally present an encapsulated condition which is hard to assess by means of usual surgical technologies. Evaluations of this type are especially critical in determining whether or not the implanted device may be explanted with a certain technology.

Presently, regarding implants in the form of leadless pacemakers, fluoroscopy techniques are frequently used to facilitate re-engagement with catheter-based delivery/explantation tooling. Such methods require the incorporation of materials and marker bands within the tooling and implant that are opaque when imaged using fluoroscopy.

Furthermore, fluoroscopy images only represent a two-dimensional representation of the in-patient hardware and anatomical features. Unfortunately, when paired with the 3-dimensional nature of e.g. a patient's heart chambers, lacking C-arm rotation and surveys of the procedural landscape from multiple viewpoints, the clinician is left without any real in-body depth perception. As such, alignment on screen via fluoroscopy procedures offers no direct means for assessing an implant/catheter offset into and out of the field of view. To survey such conditions via C-arm rotation undesirably adds to the overall duration and complexity of the surgical procedures.

The presently available solutions for managing recapture dynamics also lack any capacity for grading device encapsulation conditions.

Thus, based on the above, it is an objective to provide improved technologies for explanting an implanted implant. In particular, control of the explantation shall be improved.

A system having the features of claim 1, a system according to claim 10, a method having the features of claim 14, and a method with the features of claim 15 are provided. Further embodiments are subject matter of dependent claims.

In one aspect, a medical implant system is disclosed. The system comprises an implant that is implantable into a patient, and a catheter configured for explanting the implant. The catheter and/or the implant is (are) configured to measure a distance between a tip of the catheter and the implant. The system is configured to generate (and particularly transmit) an output signal indicative of said distance.

In another aspect, a medical implant system is disclosed. The system comprises an implant that is implantable into a patient, and a catheter which is configured for explanting the implant. The system comprises a sensor element (e.g. a unit, particularly a measuring and analyzing circuit) which is configured to measure at least one physical quantity indicative of a local anatomical environment of the implant, particularly for determining encapsulation conditions of the implant with respect to surrounding tissue. The sensor element may be configured to generate an output signal indicative of the at least one physical quantity. The sensor element may be arranged on the implant or in the implant.

In yet another aspect, a method for recapturing and/or explanting an implanted implant is provided, particularly using the system disclosed herein, wherein upon approaching the implant with the catheter, a distance between a tip of the catheter and the implant is measured using the catheter and/or the implant.

In a further aspect, a method for recapturing and/or explanting an implanted implant is disclosed, particularly using the system disclosed herein, wherein upon approaching the implant with a catheter a physical quantity indicative of a local anatomical environment of the implant is measured using the catheter and/or the implant.

The system (e.g. the catheter and/or the implant) may be configured to determine said distance in real time upon an approach of the tip of the catheter towards the implant.

Particularly, the present invention proposes the incorporation of hardware components within the delivery/explantation catheter and/or implant to facilitate the assessment of both proximity and/or interaction dynamics during device recapture procedures. Particularly, a dynamic, real-time feedback can report (either directly or indirectly) catheter-to-implant separation distances, information regarding intervening anatomical features, and the quality of catheter-to-implant engagements following successful implant recapture.

In one embodiment, a sensing element and/or a transceiver element is arranged at the implant facing the tip of the catheter, offering flexibilities for reporting proximity and connection status with the implant. The system may comprise means of the following list for measuring the distance: polled impedance or coupling capacitance measurements, implant-emergent signal-strength command relays, sonar, ultrasound, magnetic means and any combination thereof.

Within this paradigm, direct means for reporting such information may manifest implementations that are calibrated to generate a reading of the actual physical distance between the in-body catheter tip and the implant. In an embodiment, this information can be reported through a display of the system (e.g. on the catheter) or fed as an input to a fluoroscopy system being used during explantation. Such a feed can be interpreted by the fluoroscopy system to generate actual dimensional readings on the same display as that used to render 2-D depictions of in-body conditions, thus expanding the fluoroscopy system's capacity for generating depth of field insight.

Furthermore, through indirect means, the system may be configured to additionally or alternatively provide feedback through either generated vibration responses within the handle of the catheter, auditory feedback such as more frequently spaced sound signals dependent upon hardware proximity, colormetric shading of the background hue of the affiliated fluoroscopy system, or other similar approaches.

Particularly, said output signal may offer guidance on the potential for viable catheter/implant engagement as well as updates on the catheter tip and implant interaction quality both prior to and following engagement. Reporting of the quality of the catheter-to-implant engagement following successful recapture dynamics provides an added confidence to the attending clinician in terms of an interpretation of the reliable mechanical connectivity emergent between the implant and the catheter. Such confidence adds to an overarching patient safety profile that helps ensure that subsequent attempts to separate the implant from the patient (e.g. the myocardium) are conducted under best case conditions where the implant is unlikely to become uncontrollably dislodged and migrate freely within the patient's circulatory system.

Some embodiments can represent designs wherein elements at the proximal end of the implant either physically touch and/or become capacitively coupled (due to near proximity) to elements at the distal end of the catheter system. Monitoring this type of engagement could represent a binary response where conduction, coupling, impedance or other measurable values exceed a threshold known to represent robust catheter to implant engagement and, in turn, provide a "thumbs up" condition indicative of sufficient mechanical engagement. Other approaches could simply provide the number associated with any of these measurable quantities and, in turn, report the metric allowing the attending clinician to judge whether or not the observed values were "good enough" to grade catheter/implant engagements as robust. Such measurements could be performed between discrete points on the tip of the catheter and the back of the implant. The discrete points could embody multiple spots on the back end of the implant wherein engagement with a calibrated subset of these points on the implant and catheter proved essential for establishing confidence in the security of their engagement. Further, the use of spot electrode/sensor measurements could instead be avoided by taking a bulk-average approach wherein a sensing capability at the end of the catheter could be used to grossly assess near-field interactions with the implant emergent from metrics that change simply by the present or absence of the implant at the sensor. Such assessments offer the option for being passive or active. In the case of passive measurements a sense channel examines the raw changes in received input whereas active strategies would transmit an output and monitor for the response generated by the presence or absence of the implant (e.g. sonar). Assessment of engagement can be driven/conducted by the catheter itself or by the implant. In the case of implant-based measurements the findings would need to be relayed to the outside world in some manner which could involve command relay to the catheter which in turn passes said information to a user interface or through other monitoring inclusive of inductive coil-based communications or conducted galvanic (Z-comm) dialogue with an external (outside of patient) device.

The catheter and/or the implant may be configured to measure said distance continuously, periodically, or at a user-instated frequency.

The catheter may comprise a measuring unit which is configured to interact with the implant for measuring the distance. Alternatively, another measuring unit configured to interact with the catheter (or the catheter tip) for measuring the distance may be arranged in the implant or on the implant.

The measuring unit (or the other measuring unit) may be configured to generate said output signal.

The implant may comprise a transceiver unit configured to receive call signals from the measuring unit and to transmit response signals to the measuring unit for measuring said distance.

The implant may comprise a beacon for transmitting a beacon signal towards the measuring unit. The measuring unit may be configured to receive said beacon signal and to determine said distance using the received beacon signal.

The implant may be an intracardiac pacing system (e.g. a leadless pacemaker). The intracardiac pacing system may be implantable inside a heart of a patient, e.g. in the right or left ventricle, or in the right or left atrium. The system may be configured to locate a (non-pacing) terminus of an implanted intracardiac pacing system.

The system may be configured to transform said output signal that is indicative of said distance into one of: a haptic signal, a vibration, an acoustic signal (e.g. beeping or similar audible output), an optical signal, a visual output (e.g. dynamic coloring of content visible on the fluoroscopy system used in the implantation facility), and numeric data (e.g. dynamic updating of catheter-to-implant distance measurements).

Particularly, the catheter may comprise a signal device configured for transmitting the transformed signal (e.g. haptic signal, acoustic signal, optical signal) to the user such as a vibrator, a loud speaker or a display. The signal device may be arranged in a handle of the catheter, e.g. a vibrating handle. Further, the system may comprise a display connected to the catheter for showing said optical signal or visual output (e.g. to a physician or user of the catheter).

Furthermore, to grade implant encapsulation conditions, the system can be configured to survey signal-based feedback passed between the implant and the catheter, e.g. via near-field, beacon-like relay. Signal transit delay measurements, calibrated attenuations dynamics, and other similar effects would service assessments of this type. Alternatively, periodic pacing impedance measurements native to the implant itself could monitor encapsulation conditions.

The sensor element of the system/implant can be used to subsequently assess the clinical feasibility of recapture and explantation procedures for the implant, wherein particularly such assessments may employ a component arranged solely on the catheter, components arranged on the catheter and the implant, or components solely arranged on the implant.

Particularly, the present invention includes the use of physical layer measurement means (e.g. by the sensor element) that may embody impedance-based measurements (inclusive of those that are purely resistive, purely capacitive, purely inductive, as well as all relevant/feasible combinations), acoustic/sonar-based methods (whether reflective akin to submarine "pings" or employed as a vehicle for communication dialogues), galvanic methods, and electromagnetic coupling strategies.

The implant may be configured to store said at least one physical quantity or data derived therefrom, e.g. in memory.

Further, in an embodiment, the implant may be configured to make said at least one quantity/data available for export to other devices or to the user/physician thereby particularly enabling encapsulation assessments prior to any need to introduce catheters within the patient vasculature. The implant may be configured to report such effects as part of standard interrogation data and/or as a component of frequently (e.g. daily, weekly or monthly) sent data. Furthermore, home monitoring reliant strategies especially could serve to inform the clinician as to whether or not groin-based catheterization procedures should be used at all when contemplating "change out" type use cases. In effect, such metrics could inform the clinician as to whether the standard delivery/explantation catheter would prove likely to support successful device recapture and removal or if alternative approaches might prove necessary - even prior to accessing patient vasculature.

The system may comprise a remote control (or some other suitable external device) configured to trigger said measuring of said at least one physical quantity by the implant, particularly for purpose of determining the feasibility of implant recapture/explantation.

The features of the system referring to the distance measurement may be combined with the features of the system relating to the measurement of the physical quantity indicative of a local anatomical environment of the implant. Thus, in one embodiment, a system is provided which comprises an implant that is implantable into a patient, and a catheter configured for explanting the implant. The catheter and/or the implant is (are) configured to measure a distance between a tip of the catheter and the implant. The system is configured to generate (and particularly transmit) an output signal indicative of said distance. The system further comprises a sensor element (e.g. a unit, particularly a measuring and analyzing circuit) which is configured to measure at least one physical quantity indicative of a local anatomical environment of the implant.

In an analogue manner, the features of the method referring to the distance measurement may be combined with the features of the method relating to the measurement of the physical quantity indicative of a local anatomical environment of the implant. In one embodiment, a method for recapturing and/or explanting an implanted implant is provided, particularly using the system disclosed herein. Upon approaching the implant with the catheter, a distance between a tip of the catheter and the implant is measured using the catheter and/or the implant, and a physical quantity indicative of a local anatomical environment of the implant, particularly for determining encapsulation conditions of the implant with respect to surrounding tissue, is measured using the catheter and/or the implant.

The features of the systems disclosed herein can also be applied to the methods and vice versa.

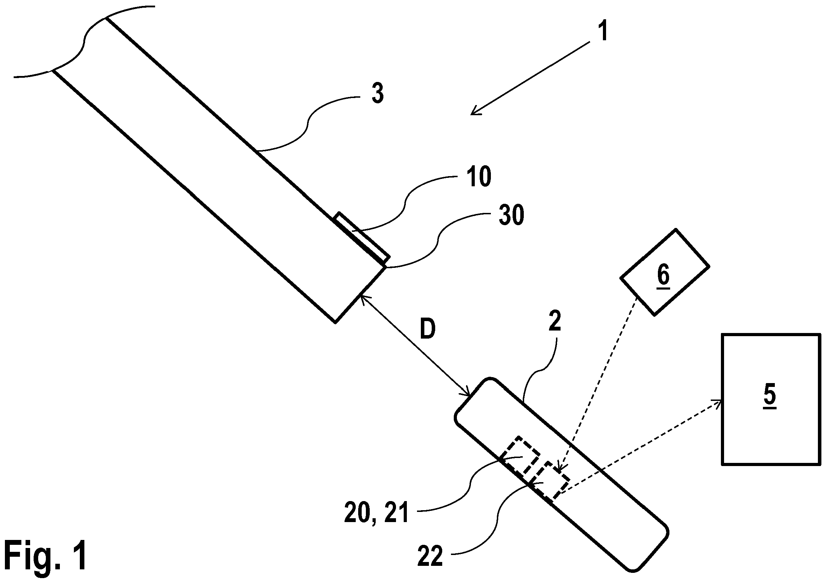

Further features and embodiments are described below with reference to a Figure, wherein
- Fig. 1: shows an embodiment of the system.

Particularly, a host of unique physical methods are available for assessing catheter-to-implant proximity. According to some embodiments, methods that also afford evaluations of both the connectivity conditions following successful recapture and potentially facilitate capabilities for reporting intervening physiological conditions are available. By leveraging a single physical method to approach this spectrum of needs, the overall footprint needed to accommodate such capabilities in a single system can be reduced.

In one embodiment, practical implementations of the complexity and hardware affiliated with these assessments of catheter/implant proximity and connection conditions resides mostly or wholly within the delivery and/or explantation tooling (e.g. the catheter), sidestepping further burdens to the implant's precious volumetric resources - especially considering that such implant-based support would only be needed during infrequent surgical interaction use cases.

Fig. 1 shows a schematic illustration of an embodiment of a medical implant system 1, comprising an implant 2 that is implantable into a patient, and a catheter 3 configured for explanting the implant 2. For example, the implant 2 is an intracardiac pacing system (e.g. a leadless cardiac pacemaker) that can be implanted into a ventricle or an atrium of the heart of the patient. Particularly, said catheter 3 can also be configured for delivering the implant 2 to the implantation site and for implanting the implant at the implantation site. The catheter 3 and/or the implant 2 is/are configured to measure a distance D between a tip 30 of the catheter 3 and the implant 2, wherein the system 1 is further configured to generate an output signal indicative of said distance D.

Particularly, for measuring said distance D, the system 1 can comprise a measuring unit 10 arranged on the catheter 3 (e.g. at the tip 30) and configured to interact with the implant 2 for measuring said distance D. Further, particularly, the measuring unit 10 can be configured to generate said output signal that is indicative of the distance D.

Particularly, in an embodiment, the implant 2 can comprise a transceiver unit 20 configured to receive call signals from the measuring unit 10 and to transmit response signals to the measuring unit for measuring said distance D. Alternatively, the implant 2 may comprises a beacon 21 for transmitting a beacon signal towards the measuring unit 10, which measuring unit 10 is configured to receive said beacon signal and to determine said distance D using the received beacon signal.

According to a further embodiment, the system 1 can comprises a sensor element 22 such as a measuring and analyzing circuit integrated into the device that is adapted for measuring at least one physical quantity indicative of the local anatomical environment of the implant, particularly for determining encapsulation conditions of the implant 2 with respect to surrounding (e.g. heart) tissue. Particularly, the implant 2 can be configured to store said at least one physical quantity or data derived therefrom and/or to transmit said at least one physical quantity or data derived therefrom to an external device 5. Further, the system 1 can comprises a remote control 6 configured to trigger said measuring of said at least one physical quantity by the implant 2.

Particularly, in an embodiment, the implant 2 can use polled impedance measurements for determining encapsulation without further crowding prevalent volumetric design considerations. Such capabilities would likely leverage the natively-supported pacing impedance measurements. Particularly, the implant 2 can monitor such a quantity for changes in values as a function of device encapsulation. Further, the implant 2 may monitor both pacing output capacitance and resistance values, in order to further improve reporting of the device encapsulation condition.

It is further conceivable that the implant/ leadless system 2 could be structured such that the implant 2 dynamically measures the pacing impedances in a polling-based fashion. The values of those measurements could be directly modulated via electromagnetic interactions with the in-chamber catheter tip 30 and relayed to external devices via implant command output. Extensions of this strategy could demand that the implant pacing output establishes a response detectable by the catheter tip 30 - potentially with electrical resonance responses designed in as a function of catheter-to-implant separation distances.

Furthermore, coupling capacitances could be used to benchmark proximity considerations. Monitoring such readings could, in turn facilitate signal-strength indicators reflective of catheter/implant nearness.

In more complex fashions, a catheter tip presenting one or more sonar and/or an ultrasound transceivers can also be used in order to support assessments of intervening physiology and device encapsulation conditions. Transit and relay of signals emitted from the tip 30 of the catheter 3 to the implant 2 and back to the catheter 3 can further be used to assess in-chamber conditions.

Simplistic formats, rather than intending to report proximity conditions between the catheter 3 and the implant 2 could simply intend to facilitate mechanical pairing of the catheter tip 30 with the non-therapeutic terminus of the implant 2. Such capabilities can be achieved through the use of an electromagnet within the tip 30 of the catheter 3 that attracts the catheter tip 30 and implant 2 to one another. Through a corresponding design of both the back end of the implant 2 and the format of the catheter tip 30 such mating could prove to be a very fluid interaction that offsets explicit demands for executing nuanced procedural skills.

The feedback of proximity and/or interaction dynamics can particularly be reported to the clinician through a fluoroscopy display used during explantation. Other approaches as described herein can also be used, particularly auditory sound signals indicative of proximity and/or connection status or even haptic feedback through the handle of the delivery/explantation tooling or catheter 3.

The systems and/or the methods may have the following advantages:
- reducing overall surgical procedure difficulty, durations, and affiliate fluoroscopy exposure;
- evaluating the extent of device encapsulation subject to chronic residence within the patient; and
- grading the quality of the engagement between the catheter and the implant prior to separating the device anchor from patient myocardium.

### List of reference numerals

- 1: system
- 2: implant
- 3: catheter
- 5: external device
- 6: remote control
- 10: measuring unit
- 20: transceiver unit
- 21: beacon
- 22: sensor element
- 30: catheter tip
- D: distance

## Claims

1. A medical implant system (1), comprising:
- an implant (2) that is implantable into a patient, and
- a catheter (3) configured for explanting the implant (2),
wherein the catheter (3) and/or the implant (2) is configured to measure a distance (D) between a tip (30) of the catheter (3) and the implant (2), and wherein the system (1) is further configured to generate an output signal indicative of said distance (D).

2. The medical implant system (1) according to claim 1, wherein the catheter (3) and/or the implant (2) is configured to measure said distance (D) continuously, periodically, or at a user-instated frequency.

3. The medical implant system (1) according to claim 1 or 2, wherein the catheter (3) comprises a measuring unit (10) configured to interact with the implant (2) for measuring said distance (D).

4. The medical implant system (1) according to claim 3, wherein the measuring unit (10) is configured to generate said output signal.

5. The medical implant system (1) according to claim 3 or 4, wherein the implant (2) comprises a transceiver unit (20) configured to receive call signals from the measuring unit (10) and to transmit response signals to the measuring unit (10) in response to the call signals for measuring said distance (D).

6. The medical implant system (1) according to one of the claim 3 to 5, wherein the implant (2) comprises a beacon (21) for transmitting a beacon signal towards the measuring unit (10), wherein the measuring unit (10) is configured to receive said beacon signal and to determine said distance (D) using the received beacon signal.

7. The medical implant system (1) according to claim 1 or 2, wherein the implant (2) comprises another measuring unit configured to interact with the catheter (3) or the tip (30) of the catheter (3) for measuring said distance (D).

8. The medical implant system (1) according to one of the preceding claims, wherein the implant (2) is an intracardiac pacing system.

9. The medical implant system (1) according to one of the preceding claims, wherein the system (1) is configured to transform said output signal into one of: a haptic signal, a vibration, an acoustic signal, an optical signal, a visual output, and numeric data.

10. A medical implant system (1), comprising:
- an implant (2) that is implantable into a patient, and
- a catheter (3) configured for explanting the implant (2),
wherein the system (1) comprises a sensor element (22) which is configured to measure at least one physical quantity indicative of a local anatomical environment of the implant (2).

11. The medical implant system (1) according to claim 10, wherein said sensor element (22) is arranged in the implant (2) or on the implant (2).

12. The medical implant system (1) according to claim 11, wherein the implant (2) is configured to store said at least one physical quantity or data derived therefrom and/or to transmit said at least one physical quantity or data derived therefrom to an external device (5).

13. The medical implant system (1) according to one of the claims 10 to 12, further comprising a remote control (6) configured to trigger said measuring of said at least one physical quantity by the sensor element (22).

14. A method for recapturing and/or explanting an implanted implant (2), particularly using a system (1) according to one of the claims 1 to 9, wherein upon approaching the implant (2) with a catheter (3) a distance (D) between a tip (30) of the catheter (3) and the implant (2) is measured using the catheter (3) and/or the implant (2).

15. A method for recapturing and/or explanting an implanted implant (2), particularly using a system (1) according to one of the claims 10 to 13, wherein upon approaching the implant (2) with a catheter (3) a physical quantity indicative of a local anatomical environment of the implant (2) is measured using the catheter (3) and/or the implant (2).
